# FASCICULE DE BREVET EUROPEEN

(11) **EP 0 564 321 B1**
(45) Date de publication et mention de la délivrance du brevet: **04.12.1996**
(21) Numéro de dépôt: 93400732.9
(22) Date de dépôt: 22.03.1993
(51) Int. Cl.: A61M 39/02, A61M 25/04

(54) **Dispositif d'injection d'un médicament**
Vorrichtung zur Einspritzung eines Medikaments
Drug injecting device

(30) Priorité: 01.04.1992 FR 9203961
(43) Date de publication de la demande: 06.10.1993
(73) Titulaire: B. BRAUN CELSA, F-86360 Chasseneuil du Poitou (FR)
(72) Inventeur: Descottes, Bernard, Prof., F-87000 Limoges (FR); Gautier, Jean-Philippe, F-86130 Jaunay Clan (FR)
(74) Mandataire: Lerner, François

(56) Documents cités:
- DE-C- 3 539 022
- US-A- 4 222 380

## Description

L'invention concerne un dispositif d'injection d'un produit pour le traitement d'une maladie, telle notamment qu'une affection ayant entraîné des métastases.

Il existe déjà des dispositifs de ce type connus comprenant :
- une capsule distributrice dudit produit traitant (habituellement sous forme liquide injectable), cette capsule étant implantable sous la peau du patient à traiter,
- et au moins un tube de diffusion, implantable dans le corps sous la peau de ce patient et communiquant avec ladite capsule pour diffuser le produit traitant.

Ainsi l'accès aux veines et artères, au moyen d'un cathéter ou tube relié à une chambre implantable sous la peau et munie d'une membrane élastique perçable, sans fuite possible, par une aiguille hypodermique d'injection est déjà connu.

Une description générale est d'ailleurs donnée au brevet US-A- 4 781 685.

Toutefois, à ce brevet, et plus généralement dans une technique souvent employée notamment pour le traitement des métastases, le tube de diffusion est installé de manière à déboucher au niveau du coeur, et plus précisément de l'oreillette droite.

Ainsi, à partir du coeur, le produit traitant est distribué dans le corps du patient avec le flux sanguin.

Or, une telle implantation du tube de diffusion au niveau du coeur peut poser certains problèmes liés, outre à la zone critique d'implantation, notamment à la dilution du produit dans le sang, donc à la quantité injectée au regard de l'efficacité obtenue.

En outre, seul est alors autorisé un traitement à distance de la zone malade, le flux sanguin constituant l'unique voie de distribution et d'approche de la zone à traiter, laquelle dans bon nombre de cas est constituée par un site solide ayant une consistance, tel qu'un organe.

Pour diversifier les voies d'accès vasculaires, il a également déjà été proposé aux praticiens d'utiliser deux cathéters de diffusion séparés pour injecter un liquide de traitement à l'intérieur d'une veine et d'une artère respectivement, avec pour chaque cas une dose appropriée de médication, les cathéters concernés pouvant être adaptés soit pour émerger, à leur extrémité proximale, à l'extérieur du corps du patient, soit pour être raccordés chacun à une dite capsule implantable.

Parmi les inconvénients à de tels dispositifs, on peut en particulier noter que :
- l'utilisation de deux cathéters associés à deux capsules implantables séparés, ou à une capsule double, est onéreuse et désagréable pour le patient qui risque de mal tolérer l'implantation,
- l'utilisation d'un nombre de cathéters, ainsi associés, largement supérieur à deux n'est pas réaliste en pratique,
- puisque de tels systèmes sont généralement prévus pour injecter des doses différentes de liquide traitant, les risques d'erreur de dosage et/ou d'inversion de cathéters doivent absolument être évités, ce qui implique la prévision de moyens de sécurité complémentaires appropriés,
- si finalement seul un unique cathéter est utilisé, le nombre de voies d'accès à la zone du corps à traiter est nécessairement limité (deux au plus : la voie veineuse et le voie artérielle), la dose de produit traitant à injecter ne pouvant donc varier que dans des proportions limitées, ce qui peut gêner le médecin traitant,
- si ce médecin ne peut utiliser que la seule voie d'accès vasculaire, le liquide injecté est nécessairement dilué dans le sang et est donc moins efficace.

Un objet de l'invention est de remédier à de tels problèmes.

Pour cela, il est tout d'abord proposé que le cathéter utilisable pour injecter le produit traitant dans le corps du patient présente, à une distance déterminée de son extrémité proximale, une ou plusieurs ramifications.

En particulier, ce cathéter pourra présenter, du côté de cette extrémité proximale (extrémité à partir de laquelle le liquide traitant est injecté), une branche unique se ramifiant ensuite en pouvant présenter une forme arborescente.

Ainsi, un traitement "par irrigation" plus au moins étendu suivant le nombre de ramifications, sera possible, en substitution ou en complément à la voie d'accès sanguine.

Dans le premier cas, si par un exemple un site de dimensions importantes et pouvant être gravement atteint doit être traité (par exemple un gros organe), toutes les ramifications terminales du tube de diffusion situées du côté de l'extrémité distale de ce dernier pourront alors être équipées de moyens de fixation, pour pouvoir injecter le produit de traitement en différents endroits judicieusement choisis du site, la longueur du cathéter étant alors adaptée en conséquence.

Si par contre un complément de traitement par voie sanguine s'avère approprié, certaines de ces ramifications terminales pourront être dépourvues desdits moyens de fixation, notamment pour pouvoir être alors introduites en milieu sanguin (par exemple à proximité du foie si celui-ci doit être traité) en y diffusant le produit du traitement.

Un troisième cas peut encore se produire, cas dans lequel un traitement uniquement par voie sanguine est préféré, le traitement pouvant alors s'effectuer à partir d'un cathéter unique, par différents vaisseaux (veines et artères) pour multiplier les voies d'accès à la zone à traiter. Les ramifications terminales du tube dépourvues de moyen de fixation seront alors coupées à mesure et introduites par voie chirurgicale.

Un autre objet de l'invention est de proposer une solution qui, en tenant compte des problèmes évoqués ci-dessus, va permettre au praticien de pouvoir à volonté :
- injecter si nécessaire le produit traitant "directement" à l'endroit ou à proximité de l'endroit à traiter, en particulier dans le cas du traitement d'un site solide tel qu'un organe (exemple : métastases du foie),
- et injecter ainsi la médication sans passer nécessairement par la voie sanguine.

Pour obtenir ces effets, on prévoit que la longueur du cathéter sera adaptée pour qu'il se prolonge jusqu'au site d'implantation et, de façon préférentielle, qu'il puisse s'y fixer, de préférence de manière séparable, tout en autorisant bien entendu la diffusion du produit à cet endroit.

Ainsi, le praticien pourra disposer d'un système d'infiltration pour un traitement loco-régional.

S'il s'avère préférable d'effectuer une infiltration profonde dans le site, les moyens de fixation en question du tube pourront notamment comprendre un crochet d'extrémité assujetti au tube pour le prolonger, le crochet étant percé à la manière d'une aiguille pour pouvoir pénétrer dans le site et être ainsi apte à y injecter en profondeur le produit de traitement.

On a également imaginé une solution à hélice d'accrochage et/ou aiguille d'injection.

Une description plus détaillée de l'invention va suivre, faite en référence aux dessins annexés donnés uniquement à titre d'exemples et dans lesquels :
la figure 1 montre une vue schématique d'un dispositif d'injection conforme à l'invention,
la figure 2 montre schématiquement, en vue de côté, la possible constitution de la chambre implantable,
la figure 3 montre une variante de réalisation du dispositif de la figure 1, dans le cas où le tube de diffusion présente des ramifications lui donnant une forme arborescente,
la figure 4 montre un premier mode de réalisation d'un raccord permettant la ramification du tube d'injection,
la figure 5 présente une variante de réalisation d'un tel raccord,
la figure 6 montre un exemple possible d'implantation du dispositif de l'invention dans le corps d'un patient,
la figure 7 montre la conformation de l'extrémité libre du tube de diffusion ou de l'une de ses ramifications, équipée d'une hélice de fixation,
la figure 8 est une vue en coupe le long de la flèche VIII-VIII de la figure 7,
la figure 9 montre une variante de réalisation dans laquelle l'hélice a été remplacée par un crochet de fixation et d'infiltration,
la figure 10 montre en coupe schématique agrandie, une autre variante de réalisation de l'extrémité du tube d'injection,
et les figures 11 et 12 présentent la constitution globale et le principe de fonctionnement d'une vanne implantable utilisable sur le dispositif de l'invention pour sélectionner les voies d'accès au site à traiter.

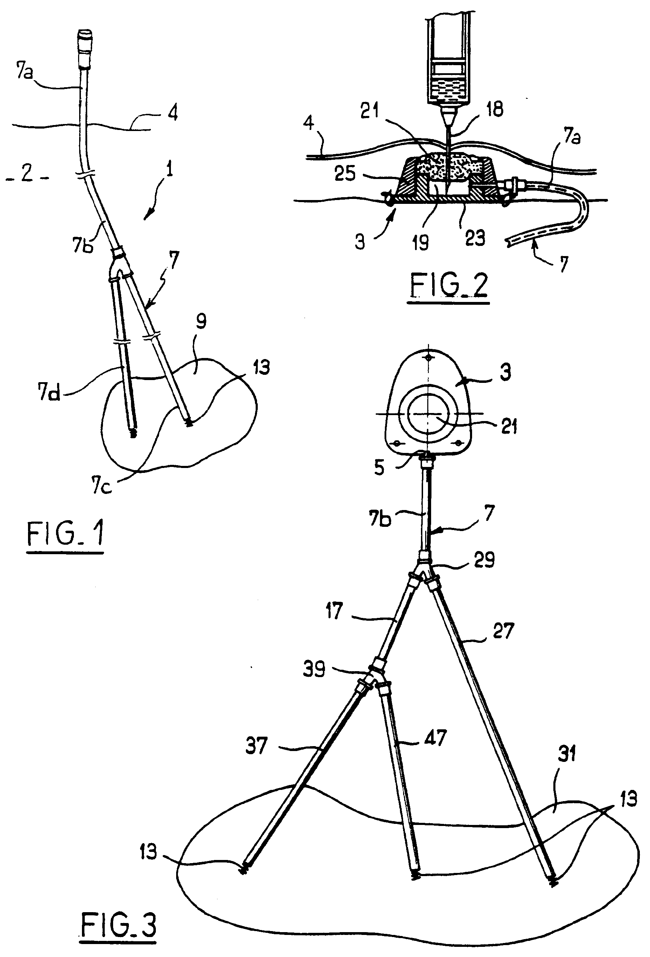

Sur la figure 1 tout d'abord, on voit donc illustré un dispositif 1 pour diffuser un médicament liquide injectable à l'intérieur du corps 2 d'un patient, à travers la surface de sa peau 4 (voire pour effectuer une ponction).

Selon un mode de réalisation de l'invention, le dispositif 1 comprend un cathéter 7 consistant en un tube flexible avantageusement réalisé en une matière sécable, biocompatible, tel que du silicone ou du polyuréthane.

Ce cathéter qui peut présenter une section constante avec un diamètre extérieur de l'ordre de 2,8 mm environ et un diamètre intérieur de l'ordre du millimètre, présente sur la figure 1 une extrémité proximale 7a émergeant à l'extérieur du corps du patient. L'extrémité 7a est ici adaptée pour être connectée à une source de liquide traitant (non représentée).

Du côté de cette extrémité proximale, le cathéter 7 se présente comme un tube unique 7b (ou branche principale) qui se divise ensuite en deux ramifications (ou sous-branches) 7c, 7d s'étendant continument jusqu'au site 9 du corps à traiter, à l'endroit duquel ces ramifications se terminent ici chacune par un moyen 13 de maintien ou de fixation au site, lequel est en l'espèce constitué par un organe ou une chair ayant une certaine consistance permettant une fixation fiable du tube à son niveau.

Sur la figure 2, on a représenté schématiquement une forme possible de réalisation d'une capsule 3 implantable à laquelle peut être connectée l'extrémité proximale raccourcie du cathéter 7, si le praticien préfère que ce dernier n'émerge pas hors du corps du patient. Tel qu'illustrée, la capsule 3 est ici implantée sous la peau 4 et l'on a repéré 18 une aiguille adaptée sur tout système approprié d'injection et/ou de ponction utilisable, dans le premier cas, pour injecter le médicament ou produit de traitement.

Fermant son réservoir interne 19, la chambre 3 comprend une paroi 21 perforable auto-obturante d'un type en soi connu, par exemple en matériau plastique siliconé.

Le fond 23 de la capsule peut être métallique, le reste de cette capsule, et notamment sa paroi latérale 25, pouvant être en matériau isolant électrique, tel qu'un plastique de qualité appropriée.

Le volume 19 communique bien entendu avec le tube 7.

Pour tout détail complémentaire de réalisation en particulier de cette capsule 3, on pourra se reporter au brevet précité US-A-4 781 685.

Sur la figure 3, on retrouve la chambre implantée 3, mais le tube de diffusion 7, au lieu de ne comprendre que deux sous-branches, en comporte quatre 17, 27, 37, 47.

En l'espèce, ces branches sont réparties en deux zones de ramifications 29, 39 donnant au cathéter une forme arborescente se prolongeant bien entendu toujours jusqu'à la zone 31 à traiter qui va pouvoir ainsi recevoir le médicament à partir des trois zones d'injection que forment les trois ramifications terminales 27, 37 et 47 du tube 7, équipées ici chacune de leur moyen de fixation d'extrémité 13.

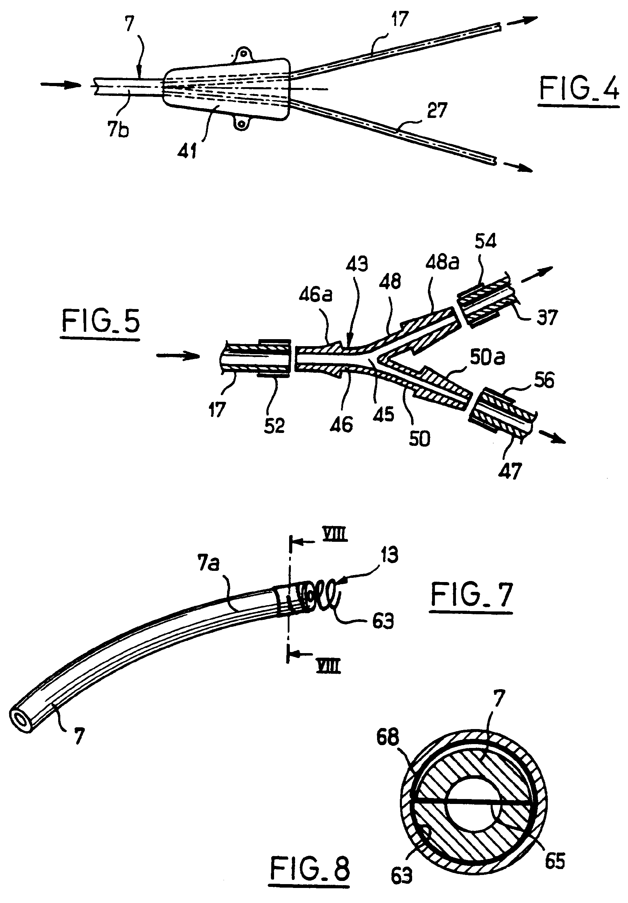

Sur les figures 4 et 5, on a illustré deux exemples possibles de réalisation des zones de ramification.

Sur la figure 4 tout d'abord, la zone illustrée comprend un raccord en "Y" pouvant par exemple constituer la zone de ramification 29 de la figure 3.

Le raccord 41 pourra se présenter comme un bloc en silicone étanche, traversé par le tube puis ses ramifications autour de la zone de raccordement desquels le raccord a été moulé.

Ainsi, le tube 7 pourra, depuis la chambre 3 et jusqu'à son extrémité distale pourvue des moyens d'accrochage 13, présenter une continuité avec, au niveau de chaque ramification ou raccord 41, une subdivision d'une branche principale telle que 7b en au moins deux sous-branches telles que 17, 27 éventuellement de section inférieure à la branche dont elles sont issues.

La solution de la figure 5 est quelque peu différente, en ce que le raccord, ici repéré 43, comporte trois branches 46, 48, 50 en "Y" percées chacune d'un canal central communiquant entre eux, en 45, le sens du flux ayant été représenté par des flèches.

Pour jouer leur rôle, chacune des branches présente extérieurement une tête de raccordement tronconique 46a, 48a, 50a, adaptées pour venir s'engager de manière étanche au fluide dans trois branches du tube 7 constituées en l'espèce par les ramifications 17, 37 et 47, le maintien en verrouillage étant complété par les bagues 52, 54 et 56.

Ainsi, dans ce cas, le tube sera au moins localement réalisé sous la forme de tronçons connectables aux branches du raccord 43.

Bien entendu, les sections respectives des pièces seront adaptées en fonction des besoins.

Sur la figure 6, une forme de réalisation du dispositif comparable à l'illustration de la figure 3 a été représentée implantée dans le corps du patient, de manière à assurer le traitement de son foie schématisé en 31.

Sur la figure, on reconnait la chambre implantable 3 de laquelle part le tube de diffusion 7 présentant en l'espèce diverses ramifications et sous-ramifications 17, 27, 37,..., 67 avec quatre ramifications terminales 37, 47, 57, 67 dont trois 47, 57, 67 sont terminées par leur moyen 13 de fixation, la quatrième ramification terminale 37 ayant en l'espèce été coupée avant son extrémité libre de manière à être dépourvue de moyens de fixation, afin de pouvoir être introduite dans le vaisseau 61.

La mise en place d'un tel dispositif s'opèrera par voie chirurgicale.

Dans l'exemple envisagé, il est conseillé de procéder comme suit, le tube 7 ayant été prévu comme constitué par une succession de tronçons sécables connectables à des raccords du type 43 de la figure 5.

On incisera tout d'abord le corps du patient à la hauteur de l'organe, de manière à y accrocher les trois moyens de fixation 13 des ramifications terminales 47, 57, 67.

Parallèlement, le cathéter 37 aura pu être partiellement glissé dans le vaisseau 61, après une légère incision et via un tube introducteur (non représenté).

Après avoir mis à longueur les cathéters, on viendra les connecter aux deux raccords 43₁, 43₂ représentés, de même pour les deux ramifications secondaires 17, 27 avec le troisième raccord 43₃, les raccords pouvant ensuite être maintenus sur les côtes à l'aide de fils de suture.

Le dernier tronçon primaire 7b du tube, connecté à l'entrée du raccord 43₃, sera alors fixé à un "tunneliseur" (en soi connu) qui se présente sous la forme d'une aiguille d'un diamètre très légèrement supérieur à celui du tube à entraîner qui est fixé à l'extrémité arrière de l'aiguille.

Une fois en place, cette aiguille pourra être poussée à travers les tissus sous-cutanés en direction de la chambre 3 laquelle aura été préalablement implantée, par exemple au niveau de la partie pré-thoracique, à l'endroit d'une logette ou la chambre sera suturée.

Après quoi, il suffira de venir brancher à cette chambre le tronçon 7b. Les incisions seront alors refermées.

Sur les figures 7 et 8, un premier mode de réalisation des moyens de fixation 13 a été illustré.

Le tronçon de tube 7 est ici équipé, à son extrémité libre 7a, d'une hélice ou vis d'accrochage 63.

L'hélice s'étend sensiblement coaxialement au tube sans gêner la diffusion du produit à injecter, en étant assujettie au tube par sa partie 65 qui traverse transversalement ledit tube.

Un manchon d'étanchéité 68, par exemple en silicone, entoure à cet endroit l'extrémité du tube et l'hélice pour compléter la fixation.

L'hélice, qui pourra être métallique, devrait pouvoir être fixée au site choisi après deux ou trois tours.

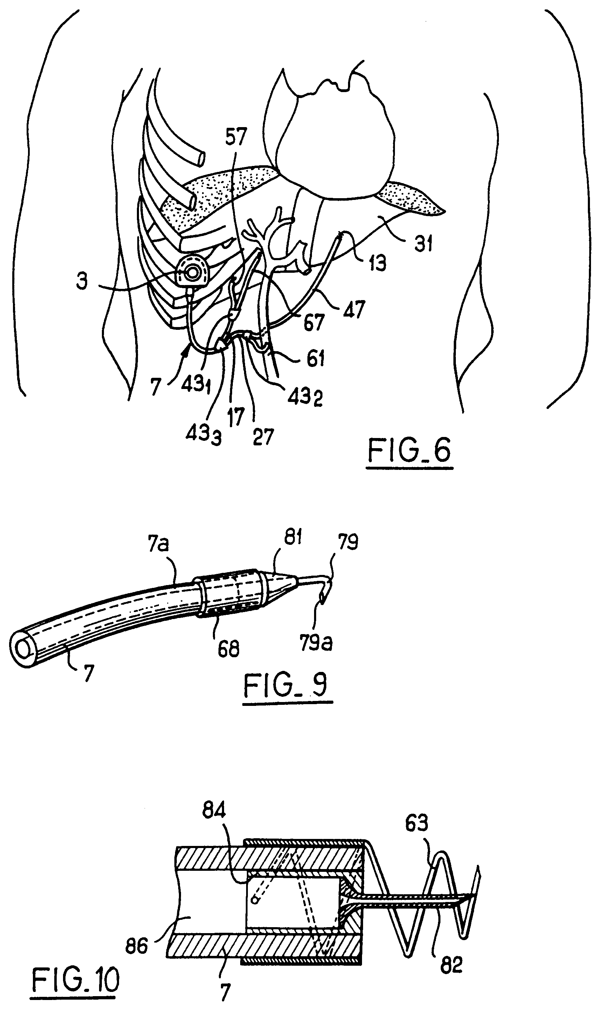

Sur la figure 9, l'hélice 63 a été remplacée par une pièce courbée, pouvant être en crochet, 79 raccordée à un embout 81 venant s'adapter étroitement autour de l'extrémité 7a du tube 7 illustré, un manchon silicone 68 venant toujours verrouiller le tout.

De préférence, l'embout 81 et le crochet 79 seront traversés par des canaux d'injection communiquant entre eux et avec le tube.

En outre, l'extrémité du crochet se terminera par une partie effilée 79a favorable à sa pénétration relativement profonde dans le site d'implantation, de manière à être alors en mesure d'y injecter "en profondeur" le produit traitant.

Sur la figure 10, l'hélice d'accrochage 63 de la figure 7 a été maintenue. Mais on lui a adjoint une aiguille centrale d'injection, rectiligne, 82 telle qu'une aiguille hypodermique ou de type "HUBER", fixée ou maintenue par une bague biocompatible, par exemple métallique, 84 à l'intérieur de l'extrémité débouchante du canal interne 86 du tube 7.

L'aiguille 82 pouvant s'étendre au milieu de l'hélice 63, il sera ainsi possible, après fixation de l'hélice, d'injecter si nécessaire en profondeur le médicament.

A la limite, l'aiguille 82 pourrait être utilisée seule, sans hélice. Dans ce cas, l'aiguille pourrait éventuellement présenter des barbes extérieures pour favoriser sur maintien en position (variante non représentée).

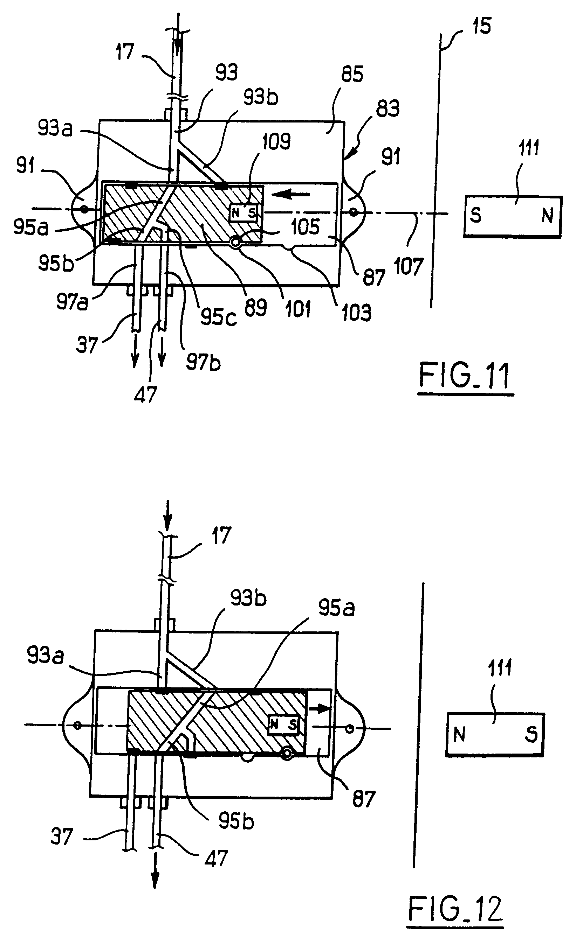

Sur les figures 11 et 12, on a représenté une vanne de dérivation ou "bypass" répérée dans son ensemble 83 et pouvant être implantée à la place de l'un au moins des raccords tels que 29, 39.

Il s'agit là d'un implant suturé sous la peau et implanté peu profond, adapté pour être commandé depuis l'extérieur, sans procédé sanglant.

En l'espèce, la vanne 83 comprend une enveloppe de protection biocompatible, par exemple en résine époxy, 85, définissant localement une cavité intérieure 87 où peut se déplacer, par exemple en translation, un moyen de dérivation 89 constitué en l'espèce par un piston aimanté.

Sur l'enveloppe 85, sont prévues des oreilles de fixation 91 afin de pouvoir si nécessaire suturer l'ensemble.

Dans cette enveloppe et dans le piston 89 sont ménagés des canaux avec différentes dérivations.

En entrée, l'enveloppe 85 présente un raccordement 93 connectable à l'une des branches ou l'un des tronçons du tube de distribution, tel qu'en l'espèce le tronçon amont 17.

Bien entendu, plusieurs entrées parallèles pourraient être prévues.

Dans l'exemple présenté, le canal 93 se divise à l'intérieur de l'enveloppe 85 en deux branches 93a, 93b débouchant sur le volume de la cavité 87.

Le moyen de dérivation 89 présente un canal complémentaire avec une branche d'entrée 95a et deux branches de sortie 95b, 95c adaptées pour pouvoir, dans une position, communiquer avec deux canaux de sortie 97a, 97b de l'enveloppe raccordés chacun à des tronçons de tubes, tels que 37 et 47 en l'espèce.

Ainsi conformée, la vanne 83 fonctionne comme suit le moyen de dérivation 89 est mobile en translation dans la cavité 87 entre deux positions stables, la stabilité étant assurée par un système à deux encoches 101, 103 et à bille 105.

Dans la première position (figure 11), le produit provenant du tronçon de tube 17 passe par la branche 93a et est distribué vers l'un et l'autre des tronçons 37, 47, via les deux branches de dérivation 95b, 95c, l'extrémité de la branche d'entrée 93b étant obturée de façon étanche par un joint qui se présente en regard sur la paroi du piston 89 compte tenu de la position que ce dernier occupe alors.

Pour assurer le déplacement du piston vers son autre position, on pourra notamment prévoir de le disposer de manière que son axe de translation 107 soit sensiblement perpendiculaire à la surface cutanée 15, le piston pouvant être équipé d'un aimant 109, du côté le plus proche de la surface de la peau.

La translation du piston sera alors obtenue depuis l'extérieur à l'aide d'un barreau aimanté 111 permettant, suivant la disposition des pôles, de repousser ou d'attirer le piston.

S'il est attiré (voir figure 12), le piston 89 occupera alors sa position seconde illustrée dans laquelle la branche 93a est obturée, tandis que la branche 93b communique avec le canal 95a puis, via le canal complémentaire 97b, avec le tronçon du tube 47.

Dans ce cas, le produit injecté, arrivant par le tronçon 17 sortira donc uniquement par le tronçon 47, sans que le tronçon 37 soit alimenté.

Bien entendu, d'autres moyens qu'une commande magnétique pourraient être utilisés, tels par exemple qu'un principe utilisant la dilatation et la compression d'un gaz en fonction d'une source chaude ou froide, voire d'une énergie électromagnétique convertie en tension et appliquée à un électro-aimant.

De la même manière, il doit être clair que le nombre des entrées et des sorties prévues sur la vanne 83 sera en pratique fonction de l'application.

## Revendications

1. Dispositif d'injection (1) d'un produit dans le corps d'un patient pour le traitement d'une maladie, comprenant un cathéter (7) implantable dans ledit corps et présentant une extrémité proximale (7a) pour recevoir à travers lui l'injection dudit produit, caractérisé en ce que le cathéter (7) présente des ramifications (7c, 7d ; 17, 27, 37, 47) à une distance déterminée de sadite extrémité proximale.

2. Dispositif selon la revendication 1, caractérisé en ce que le cathéter (7) comprend un premier tronçon unique (7b) connectable, à un extrémité, à l'une des branches (46) d'un raccord (43) à branches multiples auxquelles autres branches (48, 50) sont connectables d'autres tronçons de cathéters (17, 27).

3. Dispositif selon la revendication 1, caractérisé en ce que le cathéter (7) comprend au moins un premier tronçon (17) connectable à l'entrée d'une vanne de distribution (83) implantable sous la peau du patient et au moins deux autres tronçons (37, 47) connectables en sortie de ladite vanne, laquelle comporte un moyen de dérivation (89) commandable à distance, depuis l'extérieur du corps du patient, par un moyen de commande (111), pour faire communiquer ledit premier tronçon (7) du cathéter, avec tout ou partie desdits autres tronçons (37, 47) connectés en sortie de la vanne.

4. Dispositif selon la revendication 3, caractérisé en ce que ledit moyen de dérivation comprend un aimant (109) commandable par un barreau aimanté formant son dit moyen de commande à distance.

5. Dispositif selon la revendication 1, caractérisé en ce que le cathéter (7) comporte une branche principale (7b) se scindant, à l'endroit d'un raccord (41), en deux sous-branches (17, 27) réalisant lesdites ramifications, tout en maintenant la continuité du cathéter.

6. Dispositif selon l'une quelconque des revendications précédentes, caractérisé en ce que ledit cathéter (7) comprend plusieurs zones de ramifications (29, 39 ; 43₁, 43₂, 43₃) lui donnant une forme arborescente.

7. Dispositif selon l'une quelconque des revendications précédentes, caractérisé en ce que pour injecter ledit produit sensiblement à l'endroit d'un site solide du corps ayant une consistance, tel qu'un organe, ledit cathéter (7) est adapté pour s'étendre sensiblement jusqu'audit site et comprend des moyens de fixation (13, 63, 79) pour le fixer au site.

8. Dispositif d'injection selon l'une quelconque des revendications précédentes, caractérisé en ce que ladite extrémité proximale du cathéter (7) est connectée à une capsule (3) implantable sous la peau (4) du patient et présentant une paroi perforable auto-obturante (21) accessible à une aiguille (18) à travers la peau (4) du patient, pour injecter ledit produit dans un réservoir (19) de la capsule communiquant avec ledit cathéter.

9. Dispositif selon l'une des revendications 7 ou 8, caractérisé en ce que les moyens de fixation du cathéter (7) comprennent une hélice (63) d'accrochage assujettie audit cathéter.

10. Dispositif selon l'une des revendications 7 à 9, caractérisé en ce que ledit cathéter (7) est équipé d'une aiguille d'injection à l'extrémité libre de ses ramifications.

11. Dispositif selon la revendication 7, caractérisé en ce que les moyens de fixation comprennent une pièce courbée sensiblement en crochet (79), assujettie audit cathéter (7) et percée à la manière d'une aiguille pour pouvoir pénétrer dans le site solide (9, 31) à traiter et être apte à y injecter en profondeur ledit produit de traitement.

12. Dispositif selon l'une quelconque des revendications 7 à 11, caractérisé en ce que toutes les ramifications terminales (27, 37, 47) du cathéter (7) sont équipées à leur extrémité libre desdits moyens de fixation (13, 63, 79, 82), pour pouvoir distribuer ledit produit de traitement en différents endroits judicieusement choisis dudit site solide (31).

13. Dispositif selon l'une quelconque des revendications 7 à 11, caractérisé en ce que certaines des ramifications terminales (47, 57, 67) du cathéter (7) sont équipées à leur extrémité libre desdits moyens de fixation (13), la (les) ramification(s) restante(s) (37) en étant dépourvue(s), notamment pour pouvoir être introduite(s) en milieu sanguin, de manière à être apte à y diffuser ledit produit de traitement.

## Claims

1. Device (1) for injecting a product into the body of a patient for the treatment of a disorder, comprising a catheter (7) which can be implanted in the body and which has a proximal end (7a) for receiving through the catheter the injection of the said product, characterised in that the catheter (7) has branches (7c, 7d; 17, 27, 37, 47) at a determined distance from its proximal end.

2. Device according to Claim 1, characterised in that the catheter (7) comprises a first single portion (7b) which can be connected, at one end, to one of the branches (46) of a multiple-branch connection (43), to which other branches (48, 50) other catheter portions (17, 27) can be connected.

3. Device according to Claim 1, characterised in that the catheter (7) comprises at least one first portion (17) which can be connected to the inlet of a distribution valve (83) implantable under the patient's skin and at least two other portions (37, 47) which can be connected at the outlet of the valve, which valve comprises a bypass means (89) which can be controlled from a distance, from outside the patient's body, by a control means (111), in order to cause the first portion (7) of the catheter to communicate with all or some of the other portions (37, 47) connected at the outlet of the valve.

4. Device according to Claim 3, characterised in that the bypass means comprises a magnet (109) which can be controlled by a bar magnet forming its said remote-control means.

5. Device according to Claim 1, characterised in that the catheter (7) comprises a main branch (7b) which divides, at the site of a connection (41), into two sub-branches (17, 27) forming the branch system, while maintaining the continuity of the catheter.

6. Device according to any one of the preceding claims, characterised in that the catheter (7) comprises several branching zones (29, 39; 43₁, 43₂, 43₃) giving it a tree shape.

7. Device according to any one of the preceding claims, characterised in that, in order to inject the product substantially at the position of a solid site of the body having substance, such as an organ, the catheter (7) is adapted to extend substantially as far as the said site and comprises securing means (13, 63, 79) for securing it to the site.

8. Injection device according to any one of the preceding claims, characterised in that the proximal end of the catheter (7) is connected to a capsule (3) which can be implanted under the patient's skin (4) and which has a self-sealing perforable wall (21) accessible to a needle (18) through the patient's skin (4), in order to inject the product into a reservoir (19) of the capsule communicating with the catheter.

9. Device according to either Claim 7 or Claim 8, characterised in that the securing means of the catheter (7) comprise a fastening coil (63) fixed to the catheter.

10. Device according to any one of Claims 7 to 9, characterised in that the catheter (7) is equipped with an injection needle at the free end of its branches.

11. Device according to Claim 7, characterised in that the securing means comprise a part which is bent substantially in the shape of a hook (79) and which is fastened to the catheter (7) and is hollow in the manner of a needle in order to be able to penetrate into the solid site (9, 31) to be treated and to be capable of injecting the treatment product to a great depth there.

12. Device according to any one of Claims 7 to 11, characterised in that all the terminal branches (27, 37, 47) of the catheter (7) are equipped at their free end with the securing means (13, 63, 79, 82) in order to be able to distribute the treatment product at various carefully chosen places at the solid site (31).

13. Device according to any one of Claims 7 to 11, characterised in that some of the terminal branches (47, 57, 67) of the catheter (7) are equipped at their free end with the securing means (13), the remaining branches (37) not having securing means, especially so that they can be introduced into a blood medium, in such a manner as to be able to diffuse the treatment product there.

## Patentansprüche

1. Vorrichtung (1) zum Einspritzen eines Mittels in den Körper eines Patienten für die Behandlung einer Krankheit, mit einem in den Körper implanierbaren Katheter (7) mit einem proximalen Ende (7a), um quer durch ihn die Injektion des Mittels aufzunehmen, **dadurch gekennzeichnet**, daß der Katheter (7) Verzweigungen (7c, 7d; 17, 27, 37, 47) in einem bestimmten Abstand von seinem proximalen Ende aufweist.

2. Vorrichtung nach Anspruch 1, dadurch gekennzeichnet, daß der Katheter (7) einen ersten, einzigen Teilabschnitt (7b) aufweist, der an einem Ende mit einem der Anschlüsse (46) eines Verbindungsstückes (43) mit vielfachen Anschlüssen verbindbar ist, mit denen andere Anschlüsse (48, 50) anderer Katheterteilabschnitte (17, 27) verbindbar sind.

3. Vorrichtung nach Anspruch 1, dadurch gekennzeichnet, daß der Katheter (7) mindestens einen ersten mit dem Eingang eines Verteilerventils (83) verbindbaren Teilabschnitt, der unter die Haut des Patienten implantierbar ist, und mindestens zwei andere Teilabschnitte (37, 47) aufweist, die am Ausgang des Ventils verbindbar sind, welches ein Umleitungsmittel (89) aufweist, das aus der Entfernung von außerhalb des Körpers des Patienten mittels eines Steuermittels (111) steuerbar ist, um den ersten Teilabschnitt (7) des Katheters mit allen oder einem Teil der anderen Teilabschnitte (37, 47) zu verbinden, die am Ausgang des Ventils angeschlossen sind.

4. Vorrichtung nach Anspruch 3, dadurch gekennzeichnet, daß das Umleitungsmittel einen durch einen Stabmagneten steuerbaren Magneten (109) aufweist, der sein Steuermittel aus der Entfernung bildet.

5. Vorrichtung nach Anspruch 1, dadurch gekennzeichnet, daß der Katheter (7) einen Hauptanschluß (7b) aufweist, der sich an der Stelle eines Verbindungsstückes (41) in zwei Unteranschlüsse (17, 27) unter Bildung der Verzweigungen spaltet, wobei zugleich die Kontinuität des Katheters aufrecht erhalten wird.

6. Vorrichtung nach einem der vorhergehenden Ansprüche, dadurch gekennzeichnet, daß der Katheter (7) mehrere Verzweigungszonen (29, 39; 43₁, 43₂, 43₃) aufweist, die ihm eine baumartige Form geben.

7. Vorrichtung nach einem der vorhergehenden Ansprüche, dadurch gekennzeichnet, daß der Katheter (7) zum Injizieren des Mittels im wesentlichen an einer dauerhaften Stelle des Körpers mit einer Konsistenz, wie zum Beispiel ein Organ, geeignet ist, sich im wesentlichen bis zu der Stelle zu erstrecken und Befestigungsmittel (13, 63, 79), um ihn an der Stelle zu befestigen, aufweist.

8. Vorrichtung zum Einspritzen nach einem der vorhergehenden Ansprüche, dadurch gekennzeichnet, daß das proximale Ende des Katheters (7) mit einem unter die Haut (4) des Patienten implantierbaren Kapselteil (3) verbunden ist, das eine perforierbare, selbstverschließende Wand (21) aufweist, die für eine Nadel (18) quer durch die Haut (4) des Patienten zugänglich ist, um das Mittel in einen Speicher (19) der Kapsel einzuspritzen, der mit dem Katheter in Verbindung steht.

9. Vorrichtung nach einem der Ansprüche 7 oder 8, dadurch gekennzeichnet, daß die Befestigungsmittel für den Katheter (7) eine Aufhängungsschraube (63) aufweisen, die an dem Katheter befestigt ist.

10. Vorrichtung nach einem der Ansprüche 7 bis 9, dadurch gekennzeichnet, daß der Katheter (7) mit einer Injektionsnadel an dem freien Ende seiner Verzweigungen ausgestattet ist.

11. Vorrichtung nach Anspruch 7, dadurch gekennzeichnet, daß die Befestigungsmittel ein im wesentlichen als Haken (79) gebogenes Stück aufweisen, welches an dem Katheter (7) befestigt und nach Art einer Nadel durchstochen ist, um in die zu behandelnde dauerhafte Stelle (9, 31) eindringen zu können und geeignet zu sein, dort in der Tiefe das Behandlungsmittel zu injizieren.

12. Vorrichtung nach einem der Ansprüche 7 bis 11, dadurch gekennzeichnet, daß alle Endverzweigungen (27, 37, 47) des Katheters (7) an ihrem freien Ende mit Befestigungsmitteln (13, 63, 79, 82) ausgestattet sind, um das Behandlungsmittel an verschiedene, fachgemäß gewählte Orte der dauerhaften Stelle (31) zu verteilen.

13. Vorrichtung nach einem der Ansprüche 7 bis 11, dadurch gekennzeichnet, daß bestimmte der Endverzweigungen (47, 57, 67) des Katheters (7) an ihrem freien Ende mit Befestigungsmitteln (13) ausgestattet sind, wobei die verbleibende(n) Verzweigung(en) (37) diese nicht hat(haben), insbesondere um in Blutmilieu eingeführt werden zu können, so daß sie geeignet ist (sind), dort das Behandlungsmittel zu verteilen.
